# EUROPEAN PATENT SPECIFICATION

(11) **EP 0 749 417 B1**
(45) Date of publication and mention of the grant of the patent: **07.05.2003**
(21) Application number: 95913622.7
(22) Date of filing: 08.03.1995
(51) Int. Cl.: C07C 253/30, C07C 255/36, C07C 255/37, C07C 255/38, C07C 255/41, C07C 255/46, A61K 31/275

(54) **CYANO COMPOUNDS AND PREPARATIONS THEREOF**
CYANO-VERBINDUNGEN UND IHRE HERSTELLUNGEN
COMPOSES DE CYANO ET LEUR PREPARATIONS

(30) Priority: 11.03.1994 GB 9404706
(43) Date of publication of application: 27.12.1996
(60) Divisional application: 02079355.0
(73) Proprietor: SmithKline Beecham Corporation, Philadelphia, Pennsylvania (US)
(72) Inventor: BORDAS-NAGY, Joseph, Sunnyvale, CA 94086 (US); GORYCKI, Peter, Conshohocken, PA 19428 (US); WEBB, Kevin, Scott, Phoenixville, PA 19460 (US)
(74) Representative: Rutter, Keith, Dr.
(86) International application number: US9502965
(87) International publication number: WO95024381

(56) References cited:
- WO-A-93/19749
- WO-A-93/19750
- WO-A-93/19751
- CHEMICAL ABSTRACTS, Volume 120, issued 1994, CHRISTENSEN, "Cyclohexylbenzene Derivatives Useful for Treating Allergic and Inflammatory Diseases", abstract no. 120: 134020m; & WO,A,93 19747, (14 October 1993), page 29.
- CHEMICAL ABSTRACTS, Volume 120, issued 1994, CHRISTENSEN et al., "Preparation of Arylcyclohexanes Useful and for Inhibiting Production of Tumor Necrosis Factor", abstract no. 120: 163532k; & WO,A,93 19748, (14 October 1993), page 27.
- CHEMICAL ABSTRACTS, Volume 120, issued 1994, CHRISTENSEN et al., "Cyclohexylbenzones Useful for Treating Allergic or Inflammatory Diseases", abstract no. 120: 191326q; & WO,A,93 19750, (14 October 1993), page 56.

## Description

### Field of the Invention

The present invention relates to novel phenylcyclohexan-1-ylcarboxylic acids, and to pharmaceutical compositions containing these compounds.

### Background of the Invention

Bronchial asthma is a complex, multifactorial disease characterized by reversible narrowing of the airway and hyperreactivity of the respiratory tract to external stimuli. Identification of novel therapeutic agents for asthma is made difficult by the fact that multiple mediators are responsible for the development of the disease. Thus, it seems unlikely that eliminating the effects of a single mediator will have a substantial effect on all components of chronic bronchial asthma.

An alternative to the "mediator approach" is to regulate the activity of cells responsible for the pathophysiology of asthma. Cyclic AMP (cAMP, adenosine cyclic 3',5'-monophosphate) modulates the activity of most, if not all, of the cells that contribute to the pathophysiology of extrinsic (allergic) asthma. An elevation of cAMP would produce beneficial effects including: (1) airway smooth muscle relaxation, (2) inhibition of mast cell mediator release, (3) suppression of neutrophil degranulation, (4) inhibition of basophil degranulation, and (5) inhibition of monocyte and macrophage activation. Cyclic AMP has been shown to mediate cellular responses to a wide range of hormones, neurotransmitters and drugs; [Krebs Endocrinology Proceedings of the 4th International Congress Excerpta Medica, 17-29, 1973].

One potential means to regulate the activity of cells responsible for the pathophysiology of asthma is to control the intracellular levels of cyclic AMP. Cellular cAMP levels are elevated when an appropriate agonist binds to particular cell surface receptors, thereby activating adenylate cyclase to convert Mg⁺²-ATP to cAMP at an accelerated rate. The principal cellular mechanism for the inactivation of cAMP is hydrolysis of the 3'-phosphodiester bond by one or more of a family of isozymes referred to as cyclic nucleotide phosphodiesterases (cyclic nucleotide phosphodiesterase hereinafter "PDE"s). Hence, compounds that activate adenylate cyclase or inhibit phosphodiesterase should be effective in suppressing the inappropriate activation of airway smooth muscle and a wide variety of inflammatory cells.

It has been shown that a distinct PDE isozyme, PDE IV, is responsible for cAMP breakdown in airway smooth muscle and inflammatory cells. [Torphy, "Phosphodiesterase Isozymes: Potential Targets for Novel Anti-asthmatic Agents" in New Drugs for Asthma, Barnes, ed. IBC Technical Services Ltd., 1989]. Research indicates that inhibition of this enzyme not only produces airway smooth muscle relaxation, but also suppresses degranulation of mast cells, basophils and neutrophils along with inhibiting the activation of monocytes and neutrophils. The beneficial effects of PDE IV inhibition are markedly potentiated when adenylate cyclase activity of target cells is elevated by appropriate hormones or autocoids. Thus, PDE IV inhibitors would be effective in the asthmatic lung, where levels of prostaglandin E₂ and prostacyclin (both activators of adenylate cyclase) are elevated. PDE IV inhibitors offer a unique approach to the pharmacotherapy of bronchial asthma, and possess significant therapeutic advantages over agents currently on the market. The compounds of this invention have the ability to inhibit PDE IV.

The compounds of this invention also inhibit the production of TNF, a serum glycoprotein. Excessive or unregulated TNF production has been implicated in mediating or exacerbating a number of undesirable physiological conditions, such as diseases, and including rheumatoid arthritis, rheumatoid spondylitis, osteoarthritis, gouty arthritis and other arthritic conditions; sepsis, septic shock, endotoxic shock, gram negative sepsis, toxic shock syndrome, adult respiratory distress syndrome, cerebral malaria, chronic pulmonary inflammatory disease, silicosis, pulmonary sarcoidosis, bone resorption diseases, reperfusion injury, graft vs. host reaction, allograft rejections, fever and myalgias due to infection, such as influenza, cachexia secondary to infection or malignancy, human acquired immune deficiency syndrome (AIDS), cachexia secondary to AIDS, AIDS related complex (ARC), keloid formation, scar tissue formation, Crohn's disease, ulcerative colitis, or pyresis, in addition to a number of autoimmune diseases, such as multiple sclerosis, autoimmune diabetes and systemic lupus erythematosis.

AIDS results from the infection of T lymphocytes with Human Immunodeficiency Virus (HIV). At least three types or strains of HIV have been identified: HIV-1, HIV-2 and HIV-3. As a consequence of HIV infection, T-cell-mediated immunity is impaired and infected individuals manifest severe opportunistic infections and/or unusual neoplasms. HIV entry into a T lymphocyte requires prior T lymphocyte activation. Once an activated T lymphocyte has been infected with HIV, the T lymphocyte must be maintained in an activated state in order to permit HIV gene expression and/or HIV replication.

Cytokines, including TNF, are implicated in activated T-cell-mediated HIV protein expression and/or virus replication as playing a role in maintaining T lymphocyte activation. Therefore, interference with cytokine activity in an HIV-infected individual, such as by inhibition of TNF production, aids in limiting the maintenance of T cell activation, and thereby mitigates the progression of HIV infection to previously uninfected cells. When HIV infection of previously uninfected cells is diminished, a slowing or elimination of the progression of immune dysfunction caused by HIV infection results.

Monocytes, macrophages, and related cells, such as kupffer and glial cells, have also been implicated in the maintenance of HTV infection. These cells, like T cells, are targets for viral replication, where the level of viral replication is dependent upon the activation state of the cells. [See Rosenberg *et al*., The Immunopathogenesis of HIV Infection, Advances in Immunology, Vol. 57, 1989]. Monokines, such as TNF, have been shown to activate HIV replication in monocytes and/or macrophages [See Poli *et al*., Proc. Natl. Acad. Sci., 87:782-784, 1990], therefore, inhibition of monokine production or activity aids in limiting HIV progression as stated above for T cells.

TNF has also been implicated in various roles with other viral infections, such as the cytomegalovirus (CMV), influenza virus, adenovirus, and the herpes virus for similar reasons as those noted. TNF is also associated with yeast and fungal infections. Specifically *Candida albicans* has been shown to induce TNF production *in vitro* in human monocytes and natural killer cells. [See Riipi *et al*., Infection and Immunity, 58(9):2750-54, 1990; and Jafari *et al*., Journal of Infectious Diseases, 164:389-95, 1991. See also Wasan *et al*., Antimicrobial Agents and Chemotherapy, 35(10):2046-48, 1991; and Luke *et al*., Journal of Infectious Diseases, 162:211-214, 1990].

International Patent Applications Publication Numbers WO 93/19749 and WO 93/19750 (SmithKline Beecham Corporation) disclose certain cycloalkyl-substituted phenyl derivatives useful in the mediation or inhibition of phosphodiesterase IV (PDE IV) and as inhibitors of Tumor Necrosis Factor (TNF).

### Summary of the Invention

This invention relates to certain compounds of Formula I 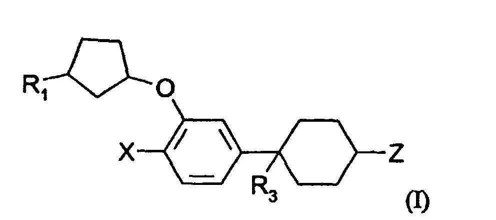 wherein;
R₁ is OH or -OCOH;
X is YR₂;
Y is O and R₂ is methyl substituted by 1 or more halogens;
Z is -C(O)OH, C(=Y')R₁₄, C(=O)OR₁₄, C(=Y')NR₁₀R₁₄, C(=NR₁₀)NR₁₀R₁₄;
R₂ is methyl or ethyl, where either methyl or ethyl may be optionally substituted by 1 or more halogens;
R₃ is CN or -C≡CH;
R₄ and R₅ are independently hydrogen or C₁₋₂ alkyl;
R₁₄ is H or R₇, or when R₁₀ and R₁₄ are as NR₁₀R₁₄, they may together with the nitrogen atom form a 5 to 7 membered ring optionally containing at least one additional heteroatom selected from O, N or S;
R₇ is -(CR₄R_{5)q}R₁₂ or C₁₋₆ alkyl wherein the R₁₂ or C₁₋₆ alkyl group is optionally substituted one or more times by C₁₋₂ alkyl optionally substituted by one to three groups selected from -F, -Br, -Cl, -NO₂, -NR₁₀R₁₁, -C(=O)R₈, -C(=O)OR₈, -OR₈, -CN, -C(=O)NR₁₀R₁₁, -OC(=O)NR₁₀R₁₁, -OC(=O)R₈, -NR₁₀C(=O)NR₁₀R₁₁, -NR₁₀C(=O)R₁₁, -NR₁₀C(=O)OR₉, -NR₁₀C(=O)R₁₃, -C(=NR₁₀)NR₁₀R₁₁, -C(=N-CN)NR₁₀R₁₁, -C(=N-CN)SR₉, -NR₁₀C(=N-CN)NR₁₀R₁₁, -NR₁₀S(=O)₂R₉, -S(O)_{m'}R₉, -NR₁₀C(=O)C(=O)NR₁₀R₁₁, -NR₁₀C(=O)C(=O)R₁₀, thiazolyl, imidazolyl, oxazolyl, pyrazolyl, triazolyl or tetrazolyl;
R₈ is -H or R₉;
R_{8'} is R₈ or fluorine;
R₉ is C₁₋₄ alkyl optionally substituted by one to three -F;
R₁₀ is OR₈, hydrogen, or C₁₋₄alkyl optionally substituted by one to three fluorines;
R₁₁ is -H or C₁₋₄ alkyl optionally substituted by one to three -F; or when R₁₀ and R₁₁ are as NR₁₀R₁₁ they may together with the nitrogen form a 5 to 7 membered ring optionally containing at least one additional heteroatom selected from O, N or S;
R₁₂ is C₃₋₇ cycloalkyl, 2-pyridyl, 3-pyridyl, 4-pyridyl, pyrimidyl, pyrazolyl, 1-imidazolyl, 2-imidazolyl, thiazolyl, triazolyl, pyrrolyl, piperazinyl, piperidinyl, morpholinyl, furanyl, 2-thienyl, 3-thienyl, 4-thiazolyl, 5-thiazolyl, quinolinyl, naphthyl or phenyl;
R₁₃ is a heterocyclic ring selected from oxazolidinyl, oxazolyl, thiazolyl, pyrazolyl, triazolyl, tetrazolyl, imidazolyl, imidazolidinyl, thiazolidinyl, isoxazolyl, oxadiazolyl or thiadiazolyl, where R₁₃ is appended to a compound of Formula (I) through a carbon atom of the heterocyclic ring, and where each heterocyclic ring may be unsubstituted or substituted by one or two C₁₋₂ alkyl groups;
m' is 0, 1 or2;
q is 0, or 2;
Y' is O or S;
or a pharmaceutically acceptable salt thereof.

This invention also relates to pharmaceutical compositions comprising a compound of Formula (I) and a pharmaceutically acceptable excipient.

Compounds of Formula (I) are useful in the treatment of additional viral infections, where such viruses are sensitive to upregulation by TNF, or will elicit TNF production *in vivo*.

### Detailed Description of the Invention

### Definitions

As used herein, the following terms and expressions have the indicated meaning.

The term "C₁₋₂ alkyl", "C₁₋₄ alkyl", or "C₁₋₆ alkyl" includes both straight or branched chain radicals of 1 to 6 carbon atoms, unless the chain length is otherwise limited thereto, including, but not limited to methyl, ethyl, *n*-propyl, isopropyl, *n*-butyl, *sec*-butyl, isobutyl, *tert*-butyl, and the like.

The term "C₃₋₇ cycloalkyl" means groups of 3-7 carbon atoms, such as cyclopropyl, cyclopropylmethyl, cyclopentyl, or cyclohexyl.

"Cytokine" means any secreted polypeptide that affects the functions of cells, and is a molecule which modulates interactions between cells in immune, inflammatory, or hematopoietic responses. A cytokine includes, but is not limited to, monokines and lymphokines regardless of which cells produce them. The cytokine inhibited by the present invention for use in the treatment of an HIV-infected human must be a cytokine which is implicated in (a) the initiation and/or maintenance of T cell activation and/or activated T cell-mediated HIV gene expression and/or replication, and/or (b) any cytokine-mediated disease associated problem such as cachexia or muscle degeneration.

"Inhibiting the production of IL-1" or "inhibiting the production of TNF" means:
a) a decrease of excessive *in vivo* IL-1 or TNF levels in a human, to normal levels or below normal levels by inhibition of the *in vivo* release of IL-1 by all cells, including but not limited to monocytes or macrophages;
b) a down regulation, at the translational or transcriptional level, of excessive *in vivo* IL-1 or TNF levels in a human, to normal levels or below normal levels; or
c) a down regulation, by inhibition of the direct synthesis of IL-1 or TNF levels as a postranslational event.

"Percentage" and "%" refers to percentage by weight of a component or ingredient based on the weight of the total composition containing such component or ingredient.

"TNF mediated disease or disease states" means any and all disease states in which TNF plays a role, either by production of TNF itself, or by TNF causing another cytokine to be released, such as but not limited to IL-1 or IL-6. A disease state in which IL-1, for instance, is a major component, and whose production or action is exacerbated or secreted in response to TNF, would therefore be considered a disease state mediated by TNF. As TNF-β (also known as lymphotoxin) has close structural homology with TNF-α (also known as cachectin), and since each induces similar biologic responses and binds to the same cellular receptor, both TNF-α and TNF-β are inhibited by the compounds of the present invention and thus are herein referred to collectively as "TNF" unless specifically delineated otherwise.

PDE IV inhibitors are useful in the treatment of a variety of allergic and inflammatory diseases, including: asthma, chronic bronchitis, atopic dermatitis, urticaria, allergic rhinitis, allergic conjunctivitis, vernal conjunctivitis, eosinophilic granuloma, psoriasis, rheumatoid arthritis, septic shock, ulcerative colitis, Crohn's disease, reperfusion injury of the myocardium and brain, chronic glomerulonephritis, endotoxic shock and adult respiratory distress syndrome. In addition, PDE IV inhibitors are useful in the treatment of diabetes insipidus and central nervous system disorders such as depression and multi-infarct dementia.

The viruses contemplated for treatment herein are those that produce TNF as a result of infection, or those which are sensitive to inhibition, such as by decreased replication, directly or indirectly, by the TNF inhibitors of Formula (I). Such viruses include, but are not limited to HIV-1, HIV-2 and HIV-3, cytomegalovirus (CMV), influenza, adenovirus and the Herpes group of viruses, such as, but not limited to, *Herpes zoster* and *Herpes simplex.*

The compounds of this invention may also be used in association with the veterinary treatment of animals, other than humans, in need of inhibition of TNF production. TNF mediated diseases for treatment, therapeutically or prophylactically, in animals include disease states such as those noted above, but in particular viral infections. Examples of such viruses include, but are not limited to feline immunodeficiency virus (FIV) or other retroviral infection such as equine infectious anemia virus, caprine arthritis virus, visna virus, maedi virus and other lentiviruses.

The compounds of this invention are also useful in treating yeast and fungal infections, where such yeast and fungi are sensitive to upregulation by TNF or will elicit TNF production *in vivo.* A preferred disease state for treatment is fungal meningitis. Additionally, a compound of Formula (I) may be administered in conjunction with other drugs of choice for systemic yeast and fungal infections. Drugs of choice for fungal infections, include but are not limited to the class of compounds called the polymycins, such as Polymycin B, the class of compounds called the imidazoles, such as clotrimazole, econazole, miconazole, and ketoconazole; the class of compounds called the triazoles, such as fluconazole, and itranazole, and the class of compounds called the Amphotericins, in particular Amphotericin B and liposomal Amphotericin B.

A compound of Formula (I) may also be used for inhibiting and/or reducing the toxicity of an anti-fungal, anti-bacterial or anti-viral agent by administering an effective amount of a compound of Formula (I) to a mammal in need of such treatment. Preferably, a compound of Formula (I) is administered for inhibiting or reducing the toxicity of the Amphotericin class of compounds, in particular Amphotericin B.

It is preferred that the R₃ group be axial and the Z group be equatorial.

The specific compounds disclosed herein are:
cis-{4-cyano-4-[3-(cis-3-hydroxycyclopentyloxy)-4-methoxyphenyl]cyclohexane-1-carboxylic acid};
cis- {-4-cyano-4-[3-(cis-3-formyloxycyclopentyloxy)-4-methoxyphenyl]cyclohexane-1-carboxylic acid}, and
cis-{-4-cyano-4-[3-(trans-3-hydroxycyclopentyloxy)-4-methoxyphenyl]cyclohexane-1-carboxylic acid}.

### Syntheses

Preparation of the compounds of Formula (I) can be carried out by one of skill in the art according to the procedures outlined reaction scheme set forth below and the specific chemistries set out in the Examples, infra. While the scheme and the examples illustrate the preparation of the cis/cis isomer(s), the cis/trans isomer(s) can be prepared by the same set of chemistries, with a nominal change in the treatment of compound 4; saponifying compound 4 provides the cis/trans compound namely the cis-{-4-cyano-4-[3-(trans-3-hydroxycyclopentyloxy)-4-methoxyphenyl]cyclohexane-1-carboxylic acid} or the corresponding compounds as defined by Formula I. The preparation of any remaining compounds of the Formula (I) not described therein may be prepared by the analogous processes disclosed herein which comprise:

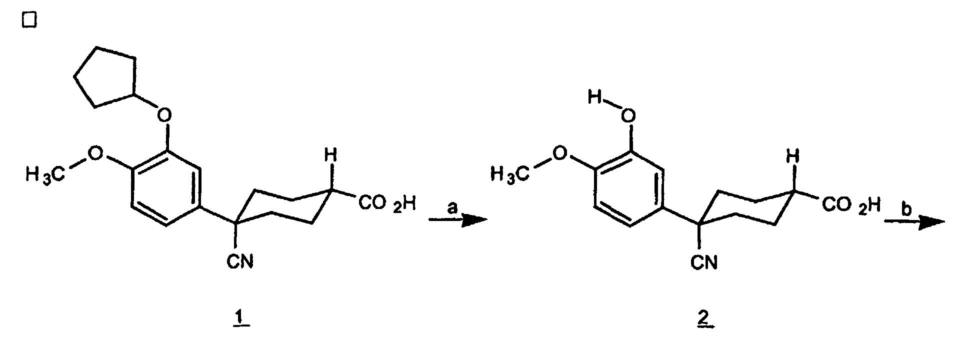

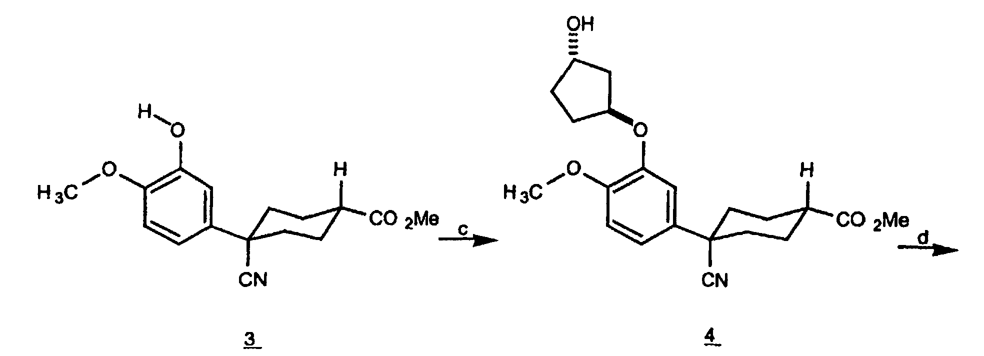

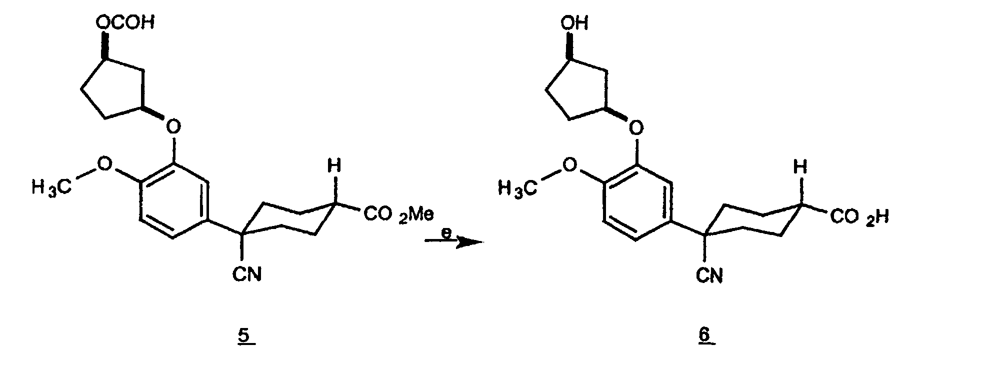

### Example 1

### Preparation of cis-[4-cyano-4-(3-cyclopentyloxy-4-methoxyphenyl)cyclohexane-1-carboxylic acid]

### 1(a) (3-Cyclopentyloxy-4-methoxyphenyl)acetonitrile

To a solution of 3-cyclopentyloxy-4-methoxybenzaldehyde (20 g, 90.8 mmol) in acetonitrile (100 mL) was added lithium bromide (15 g, 173 mmol) followed by the dropwise addition of trimethylsilylchloride (17.4 mL, 137 mmol). After 15 min, the reaction mixture was cooled to 0° C, 1,1,3,3-tetramethyldisiloxane (26.7 mL, 151 mmol) was added dropwise and the resulting mixture was allowed to warm to room temperature. After stirring for 3 h, the mixture was separated into two layers. The lower layer was removed, diluted with methylene chloride and filtered through Celite®. The filtrate was concentrated under reduced pressure, dissolved in methylene chloride and refiltered. The solvent was removed in vacuo to provide a light tan oil. To a solution of this crude a-bromo-3-cyclopentyloxy-4-methoxytoluene in dimethylformamide (160 mL) under an argon atmosphere was added sodium cyanide (10.1 g, 206 mmol) and the resulting mixture was stirred at room temperature for 18 h, then poured into cold water (600 mL) and extracted three times with ether. The organic extract was washed three times with water, once with brine and was dried (K₂CO₃). The solvent was removed in vacuo and the residue was purified by flash chromatography (silica gel, 10% ethyl acetate/hexanes) to provide an off-white solid ( m.p. 32-34° C); an additional quantity of slightly impure material also was isolated.

### 1(b) Dimethyl 4-cyano-4-(3-cyclopentyloxy-4-methoxyphenyl)pimelate

To a solution of (3-cyclopentyloxy-4-methoxyphenyl)acetonitrile (7 g, 30.3 mmol) in acetonitrile (200 mL) under an argon atmosphere was added a 40% solution of Triton-B in methanol (1.4 mL, 3.03 mmol) and the mixture was heated to reflux. Methyl acrylate (27 mL, 303 mmol) was added carefully, the reaction mixture was maintained at reflux for 5 h and then cooled. The mixture was diluted with ether, was washed once with 1N hydrochloric acid and once with brine, was dried (MgSO₄) and the solvent was removed in vacuo. The solid residue was triturated with 5% ethanol/hexane to provide a white solid (m.p. 81-82° C); an additional quantity was also obtained from the filtrate. Anal. (C₂₂H₂₉NO₆) calcd: C 65.49, H 7.25, N 3.47. found: C 65.47, H 7.11, N 3.49.

### 1(c) 2-Carbomethoxy-4-cyano-4-(3-cyclopentyloxy-4-methoxyphenyl)cyclohexan-1-one

To a suspension of sodium methoxide (350 mL, 1.55 mol, 25% w/w in methanol) in toluene (2.45 L) heated to 80° C under a nitrogen atmosphere was added a solution of dimethyl 4-cyano-4-(3-cyclopentyloxy-4-methoxyphenyl)pimelate (350.0 g, 0.87 mol) in toluene (1.05 L) over 10 min. The reaction was heated to 85° C by distilling away 250 mL of solvent and was vigorously stirred under nitrogen for 2 hours. The reaction was cooled to 50° C and was quenched with 3N (aq) HCl (700 mL, 2.1 mol). The organic layer was isolated, was washed once with deionized water (700 mL) and once with brine (700 mL). The organic layer was concentrated via low vacuum distillation to afford crude 2-carbomethoxy-4-cyano-4-(3-cyclopentyloxy-4-methoxyphenyl)cyclohexane-1-one in toluene. This was dissolved in 4.2 L of dimethyl sulfoxide and used in the next step.

### 1(d) 4-Cyano-4-(3-cyclopentyloxy-4-methoxyphenyl)cyclohexan-1-one

To a suspension of sodium chloride (315 g, 5.39 mol) and deionized water (315 mL) was added the dimethyl sulfoxide (4.2 L) solution of 2-carbomethoxy-4-cyano-4-(3-cyclopentyloxy-4-methoxyphenyl)cyclohexane-1-one ( 323 g, 0.87 mol) and the resulting suspension was heated to 155° C for 1.75 h. The reaction was cooled to 40° C, was quenched into 8 L of iced water (2° C) and was extracted with ethyl acetate (3.5 L). The aqueous layer was isolated and re-extracted with 2.5 L of ethyl acetate. The combined organic extract (6 L) was washed two times with deionized water (2 x 1 L) and once with brine (1 L). The organic layer was isolated and concentrated in vacuo to afford a residue. This residue was dissolved in refluxing isopropanol (500 mL), was cooled to 0° C and held at this temperature for 1 hour. The crystals were isolated by filtration, were washed with 250 mL of isopropanol (0° C), and were dried in a vacuum oven (45° C at 20 inches) to produce 4-cyano-4-(3-cyclopentyloxy-4-methoxyphenyl)cyclohexan-1-one. m.p. 111-112° C; Anal. (C₁₉H₂₃NO₃) calcd: C 72.82, H 7.40, N 4.47; found: C 72.72, H 7.39, N 4.48.

### 1(e) 2-[4-Cyano-4-(3-cyclopentyloxy-4-methoxyphenyl)cyclohexylidene]-1,3-dithiane

To a solution of 2-trimethylsilyl-1,3-dithiane (9.25 mL, 48.7 mmol) in dry tetrahydrofuran (80 mL) at 0° C under an argon atmosphere was added rapidly n-butyllithium (2.5M in hexanes, 19.2 mL, 48 mmol). After 10 min, the mixture was cooled to -78° C and a solution of 4-cyano-4-(3-cyclopentyloxy-4-methoxyphenyl)cyclohexan-1-one (7.53 g, 23 mmol) in tetrahydrofuran (40 mL) was added. After 10 min, aqueous sodium chloride was added, the mixture was allowed to warm to room temperature and was diluted with water. This mixture was combined with the product of three substantially similar reactions conducted on ketone (3.04, 6.01 and 6.1 g, 48.3 mmol total), the combined mixture was extracted three times with methylene chloride, the extract was dried (MgSO₄) and evaporated. Purification by flash chromatography (silica gel, 10% ethyl acetate/hexanes) provided a white solid. m.p. 115-116° C.

### 1(f) cis-[4-Cyano-4-(3-cyclopentyloxy-4-methoxyphenyl)cyclohexane-1-carboxylic acid]

To a suspension of2-[4-cyano-4-(3-cyclopentyloxy-4-methoxyphenyl)cyclohexylidene]-1,3-dithiane (140.0 g, 0.34 mol) in acetonitrile (500 mL) and deioinized water (140 mL) under nitrogen was added trifluoroacetic acid (136 g, 1.19 mol). The suspension was heated to 65° C for 1.25 h followed by the addition of 20% sodium hydroxide (420 g, 2.1 mol). The solution was heated at 70 to 75° C for an additional 1.25 h, was cooled to 45° C, deionized water (420 mL)was added followed by 3N (aq) HCl (392 mL, 1.18 mol). The suspension was cooled to 5° C and held for 1 h. The suspension was filtered, was washed with cold (5° C) deionized water ( 200 mL), and was dried in a vacuum oven (40°C at 20 inches) to obtain crude cis-[4-cyano-4-(3-cyclopentyloxy-4-methoxyphenyl)cyclohexane-1-carboxylic acid]. This material was assayed at 98.5% and was found to a 98.8:1.2 mixture of cis-to-trans isomers, which was contaminated with 0.1% of residual 1,3-propanedithiol. This material was purified via an oxidative workup as follows.

To a hot solution (65° C) of crude cis-[4-cyano-4-(3-cyclopentyloxy-4-methoxyphenyl)cyclohexane-1-carboxylic acid] (85 g, 0.247 mol) in acetonitrile (425 mL) was added 1M sodium hydroxide (425 mL, 0.425 mol). To the solution (60° C) was added 4.25 g of calcium hypochlorite and the suspension was vigorously stirred for 2 h. The reaction was concentrated by distilling out 320 mL of solvent, followed by the addition of ethyl acetate (425 mL). The reaction was again concentrated by distilling out 445 mL of solvent, was cooled to 55° C followed by the addition of ethyl acetate (1.0 L) and 6N (aq.) HCI (100 mL). The organic layer was isolated, was washed three times with deionized water (3 x 300 mL), was filtered and was concentrated by distilling out 530 mL of solvent. To the solution was added ethyl acetate (635 mL) with continued distillation to remove 750 mL of solvent. The solution was cooled to 65° C followed by the addition of hexane ( 340 mL). The suspension was cooled to 5° C, held at this temperature for 1 hour, was filtered and was washed with cold (5° C) 10% ethyl acetate/ hexane ( 200 mL). The solid was collected and was dried in a vacuum oven (40° C at 20 inches) to obtain cis-[4-cyano-4-(3-cyclopentyloxy-4-methoxyphenyl)cyclohexane-1-carboxylic acid]. This material was found to contain no trans isomer. Anal.(C₂₀H₂₅NO₄) calcd: C 69.95, H 7.34, N 4.08; found: C 69.90, H 7.35, N 4.02.

### Example 2

### Preparation of cis- {4-cyano-4-[3-(trans-3-hydroxycyclopentyloxy)-4-methoxyphenyl]-cyclohexane-1-carboxylic acid}

### 2(a) cis-[4-Cyano-4-(3-hydroxy-4-methoxyphenyl)cyclohexane-1-carboxylic acid]

To a solution of boron tribromide in dichlorormethane (0.1M, 335 mL, 33.5 mmol) under an argon atmosphere at -78° C was slowly added a solution of cis-[4-cyano-4-(3-cyclopentyloxy-4-methoxyphenyl)cyclohexane-1-carboxylic acid] (4.03 g, 11.7 mmol) in dichloromethane (180 mL). The mixture was stirred for 5 min, 15% sodium methoxide in methanol was added to pH 8-9 and the reaction was warmed to RT. Water (100mL) was added and the mixture was acidified with 3N aqueous hydrochloric acid to pH 1-2. The organic layer was separated, was dried (MgSO₄/Na₂SO₄), was filtered and was evaporated. The residue was twice dissolved in chloroform and the solution was evaporated to yield a white solid. ¹H NMR(400 MHz, CDCl₃) δ 7.01 (d, J=2.4 Hz, 1H), 6.96 (d of d, J=2.4, 8.5 Hz, 1H), 3.89 (s, 3H), 2.31 (m, 1H), 2.21 (br t, J=13.6 Hz, 4H), 1.98 (m,2H), 1.77 (m, 2H); mp 190-193° C.

### 2(b) Methyl cis-[-4-cyano-4-(3-hydroxy-4-methoxyphenyl)cyclohexane-1-carboxylate]

*p*-Toluenesulfonic acid monohydrate (0.015 g, 0.08 mmol) was added to a solution of the compound of Example 2(a) (0.70 g, 2.54 mmol) in dry methanol (20 mL) under an argon atmosphere and the reaction was stirred for 6 h at 45-50° C. The reaction was cooled to RT and was stirred for an additional 16 h. The solution was evaporated and the residue was purified by flash chromatography (silica gel, 50% hexane/ethyl acetate) to yield the title compound as a white solid. ¹H NMR(400 MHz, CDCl₃) δ 7.01 (m, 2H), 6.85 (d, J=9.1 Hz, 1H), 3.90 (s, 3H), 3.72 (s, 3H), 2.35 (t oft, J=3.6, 12.2 Hz, 1H), 2.14-2.25 (m, 4H), 2.00 (app q, J=13.4 Hz, 1H), 1.99 (app q, J=13.4 Hz, 1H), 1.77 (app t, J=13.4 Hz, 1H), 1.76 (app t, J=13.4 Hz, 1H); mp 106-107° C.

### 2(c) Methyl cis-{-4-cyano-4-[3-(trans-3-hydroxycyclopentyloxy)-4-methoxyphenyl]-cyclohexane-1-carboxylate}

The compound of Example 2(b) (0.69 g, 2.37 mmol) was dissolved in tetrahydrofuran (20 mL) under an argon atmosphere and was treated with triphenylphosphine (1.24 g, 4.74 mmol) and cis-1,3-cyclopentanediol (0.49 g, 4.74 mmol). Diethyl azodicarboxylate (0.83 g, 4.74 mmol) was added and the mixture was stirred at RT for 16 h. The solution was evaporated, the residue was diluted with ether and the white solid was removed by filtration. The filtrate was concentrated and the residue was purified by flash chromatography (silica gel, 50% hexane/ethyl acetate) to yield a mixture of the title compound and triphenylphosphine oxide. The mixture was diluted with ether and the white solid triphenylphosphine oxide was removed by filtration. Evaporation of the filtrate yielded the title compound as a sticky, colorless semi-solid. ¹H NMR(400 MHz, CDCl₃) δ 7.07 (d, J=2.4 Hz, 1H), 7.02 (d of d, J=2.4, 8.8 Hz, 1H), 6.87 (d, J=8.8 Hz, 1H), 4.99 (m, 1H), 4.37 (m, 1H), 3.85 (s, 3H), 3.74 (s, 3H), 3.16 (d, J=9.1 Hz, 1H), 2.39 (m, 1H), 1.88-2.25 (m, 12H), 1.80 (br t, J=13.5 Hz, 2H).

### 2(d) cis-{-4-cyano-4-[3-(trans-3-hydroxycyclopentyloxy)-4-methoxyphenyl]cyclohexane-1-carboxylic acid}

The compound of Example 2(c) (0.10 g, 0.27 mmol) was dissolved in 5:5:2 tetrahydrofuran/methanol/water (5 mL), sodium hydroxide (0.035 g, 0.88 mmol) was added and the mixture was stirred at RT for 3 h. The solvent was evaporated, the residue was partitioned between 5% aqueous NaOH and dichloromethane and the layers were separated. The aqueous layer was acidified to pH 3 with 3N aqueous hydrochloric acid and was extracted three times with 5% methanol in chloroform. The organic extracts were combined, were dried (MgSO4), filtered and evaporated. The residue was purified by flash chromatography (silica gel, 90:10:1 chloroform/methanol/water) to yield a solid which was slurried in ether, was collected by filtration and was dried in vacuo to afford the title compound. MS(CI/NH₃) m/e 377 [M + NH₃]+; ¹H NMR(400 MHz, CDCl₃) δ 7.08 (br s, 1H), 7.03 (br d, J=8.5Hz, 1H), 6.88 (d, J=8.5 Hz, 1H), 4.98 (m, 1H), 4.38 (m, 1H), 3.84 (s, 1H), 2.41 (m, 1H), 1.77-2.29 (m, 16H); Anal. (C₂₀H₂₅NO₅·0.9 H₂O) calcd: C, 63.95; H,7.19; N,3.73. found: C, 64.06; H, 6.88; N, 3.77; mp 161-163° C.

### Example 3

### Preparation of cis-{4-cyano-4-[3-(cis-3-hydroxycyclopentyloxy)-4-methoxyphenyl]-cyclohexane-1-carboxylic acid}

### 3(a) Methyl cis-{-4-cyano-4-[3-(cis-3-formyloxycyclopentyloxy)-4-methoxyphenyl]-cyclohexane-1-carboxylate}

The compound of Example 2(c) (0.68 g, 1.83 mmol) was dissolved in tetrahyrofuran (20 mL) under an argon atmosphere and was treated with triphenylphosphine ( 0.96 g, 3.66 mmol) and formic acid (0.17 g, 3.66 mmol). Diethyl azodicarboxylate (0.64 g, 3.66 mmol) was added and the mixture was stirred at RT for 16 h. The solution was evaporated, ether was added and the white solid was removed by filtration. The filtrate was concentrated and the residue was purified by flash chromatography (silica gel, 65% hexane/ethyl acetate) to yield the title compound as a clear colorless oil. ¹H NMR(400 MHz, CDCl₃) δ 8.02 (s, 1H), 7.0 (d of d, J=2.4, 8.2 Hz, 1H), 6.99 (d, J=2.4 Hz, 1 H), 6.87 (d, J=8.2 Hz, 1H), 5.48 (m, 1H), 4.95 (m, 1H), 3.84 (s, 3H), 3.72 (s, 3H), 2.31-2.40 (m, 2H), 2.13-2.28 (m, 7H), 1.96-2.06 (m, 3H), 1.74-1.87 (m, 3H).

### 3(b) cis-{-4-cyano-4-[3-(cis-3-hydroxycyclopentyloxy)-4-methoxyphenyl]cyclohexane-1-carboxylic acid}

The compound of Example 3(a) (0.52 g, 1.31 mmol) was dissolved in 5:5:2 tetrahydrofuran/methanol/water (20mL), sodium hydroxide (0.32 g, 8.0 mmol) was added and the mixture was stirred at RT for 2.5 h. The solvent was evaporated and the aqueous residue was acidified to pH 1-2 with 3N aqueous hydrochloric acid. The white solid product was collected, was washed with water and was dried in vacuo to afford the title compound as a white solid. MS(CI/NH₃) m/e 377 [M + NH3]+; 1H NMR(250 MHz, CDCl₃) δ 6.98 (m, 2H), 6.86 (d, J=8.2 Hz, 1H), 4.97 (m, 1H), 4.59 (m, 1H), 3.85 (s, 3H), 1.64-2.47 (m, 17H); mp 143-145° C.

### METHODS OF TREATMENT

In order to use a compound of Formula (I) or a pharmaceutically acceptable salt thereof may be used neat though a preferred technique is to present them with a carrier/diluent accordance with standard pharmaceutical practice. Any formulation compatible with the chosen method of delivery and the stability of the compound may be used. One skilled in the art will be able to select and prepare an acceptable formulation in accordance with standard practices in the field of the formulary arts.

The compounds of Formula (I) or may be administered orally (when active by this route), oral, intravenous, intraperitoneal, and intramuscular administration, topically, parenterally, or by inhalation in conventional dosage forms prepared by combining such agent with standard pharmaceutical carriers according to conventional procedures in an amount sufficient to produce the desired therapeutic activity.

The amount of a compound of Formula (I) required for therapeutic effect on topical administration will, of course, vary with the compound chosen, the nature and severity of the condition and the animal undergoing treatment, and is ultimately at the discretion of the physician.

The daily dosage regimen for oral administration is suitably about .001 mg/kg to 100mg/kg, preferably 0.01 mg/Kg to 40 mg/Kg, of a compound of Formula (I) or a pharmaceutically acceptable salt thereof calculated as the free base. The active ingredient may be administered from 1 to 6 times a day, sufficient to exhibit activity.

### UTILITY EXAMPLES

### Example A

### Inhibitory effect of compounds of Formula (I) on in vitro TNF production by human monocytes

The inhibitory effect of compounds of Formula (I) on *in vitro* TNF production by human monocytes may be determined by the protocol as described in Badger *et al*., EPO published Application 0 411 754 A2, February 6, 1991, and in Hanna, WO 90/15534, December 27, 1990.

### Example B

Two models of endotoxic shock have been utilized to determine *in vivo* TNF activity for the compounds of Formula (I). The protocol used in these models is described in Badger *et al*., EPO published Application 0411 754 A2, February 6, 1991, and in Hanna, WO 90/15534, December 27, 1990.

The exemplified compounds herein demonstrated a positive *in vivo* response in reducing serum levels of TNF induced by the injection of endotoxin.

### Example C

### Isolation of PDE Isozymes

The phosphodiesterase inhibitory activity and selectivity of the compounds of Formula (I) can be determined using a battery of five distinct PDE isozymes. The tissues used as sources of the different isozymes are as follows: 1) PDE Ib, porcine aorta; 2) PDE Ic, guinea-pig heart; 3) PDE III, guinea-pig heart; 4) PDE IV, human monocyte; and 5) PDE V (also called "Ia"), canine trachealis. PDEs Ia, Ib, Ic and III are partially purified using standard chromatographic techniques [Torphy and Cieslinski, Mol. Pharmacol., 37:206-214, 1990]. PDE IV is purified to kinetic homogeneity by the sequential use of anion-exchange followed by heparin-Sepharose chromatography [Torphy *et al*., J. Biol. Chem., 267:1798-1804, 1992].

Phosphodiesterase activity is assayed as described in the protocol of Torphy and Cieslinski, Mol. Pharmacol., 37:206-214, 1990. Positive IC₅₀'s in the nanomolar to µM range for compounds of the workings examples described herein for Formula (I) have been demonstrated.

### Example D

The ability of selected PDE IV inhibitors to increase cAMP accumulation in intact tissues is assessed using U-937 cells, a human monocyte cell line that has been shown to contain a large amount of PDE IV. To assess the activity of PDE IV inhibition in intact cells, nondifferentiated U-937 cells (approximately 10⁵ cells/reaction tube) were incubated with various concentrations (0.01-1000 µM) of PDE inhibitors for one minute and 1µM prostaglandin E2 for an additional four minutes. Five minutes after initiating the reaction, cells were lysed by the addition of 17.5% perchloric acid, the pH was neutralized by the addition of 1M potassium carbonate and cAMP content was assessed by RIA. A general protocol for this assay is described in Brooker *et al*., Radioimmunassay of cyclic AMP and cyclic GMP., Adv. Cyclic Nucleotide Res., 10:1-33, 1979. The compounds of the working examples as described herein for Formula (I) have demonstrated a positive EC₅₀s in the µM range in the above assay.

No toxic effects are expected when these compounds are administered in accordance with the present invention.

## Claims

1. A compound of Formula I 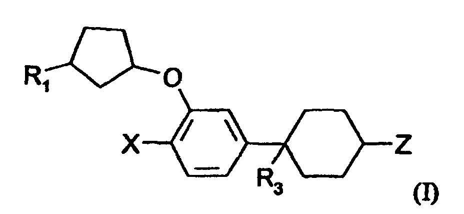 wherein;
R₁ is OH or -OCOH;
X is YR₂;
Y is O and R₂ is methyl substituted by 1 or more halogens;
Z is -C(O)OH, C(=Y')R₁₄, C(=O)OR₁₄, C(=Y')NR₁₀R₁₄, C(=NR₁₀)NR₁₀R₁₄;
R₂ is methyl or ethyl, where either methyl or ethyl may be optionally substituted by 1 or more halogens;
R₃ is CN or -C≡CH;
R₄ and R₅ are independently hydrogen or C₁₋₂ alkyl;
R₁₄ is H or R₇, or when R₁₀ and R₁₄ are as NR₁₀R₁₄, they may together with the nitrogen atom form a 5 to 7 membered ring optionally containing at least one additional heteroatom selected from O, N or S;
R₇ is -(CR₄R_{5)q}R₁₂ or C₁₋₆ alkyl wherein the R₁₂ or C₁₋₆ alkyl group is optionally substituted one or more times by C₁₋₂ alkyl optionally substituted by one to three groups selected from -F, -Br, -Cl, -NO₂, -NR₁₀R₁₁, -C(=O)R₈, -C(=O)OR₈, -OR₈, -CN, -C(=O)NR₁₀R₁₁, -OC(=O)NR₁₀R₁₁, -OC(=O)R₈, -NR₁₀C(=O)NR₁₀R₁₁, -NR₁₀C(=O)R₁₁, -NR₁₀C(=O)OR₉, -NR₁₀C(=O)R₁₃, -C(=NR₁₀)NR₁₀R₁₁, -C(=N-CN)NR₁₀R₁₁, -C(=N-CN)SR₉, -NR₁₀C(=N-CN)NR₁₀R₁₁, -NR₁₀S(=O)₂R₉, -S(O)_{m'}R₉, -NR₁₀C(=O)C(=O)NR₁₀R₁₁, -NR₁₀C(=O)C(=O)R₁₀, thiazolyl, imidazolyl, oxazolyl, pyrazolyl, triazolyl or tetrazolyl;
R₈ is -H or R₉;
R₉ is C₁₋₄ alkyl optionally substituted by one to three -F;
R₁₀ is OR₈, hydrogen, or C₁₋₄alkyl optionally substituted by one to three fluorines;
R₁₁ is -H or C₁₋₄ alkyl optionally substituted by one to three -F; or when R₁₀ and R₁₁ are as NR₁₀R₁₁ they may together with the nitrogen form a 5 to 7 membered ring optionally containing at least one additional heteroatom selected from O, N or S;
R₁₂ is C₃₋₇ cycloalkyl, 2-pyridyl, 3-pyridyl, 4-pyridyl, pyrimidyl, pyrazolyl, 1-imidazolyl, 2-imidazolyl, thiazolyl, triazolyl, pyrrolyl, piperazinyl, piperidinyl, morpholinyl, furanyl, 2-thienyl, 3-thienyl, 4-thiazolyl, 5-thiazolyl, quinolinyl, naphthyl or phenyl;
R₁₃ is a heterocyclic ring selected from oxazolidinyl, oxazolyl, thiazolyl, pyrazolyl, triazolyl, tetrazolyl, imidazolyl, imidazolidinyl, thiazolidinyl, isoxazolyl, oxadiazolyl or thiadiazolyl, where R₁₃ is appended to a compound of Formula (I) through a carbon atom of the heterocyclic ring, and where each heterocyclic ring may be unsubstituted or substituted by one or two C₁₋₂ alkyl groups;
m' is 0, 1 or 2;
q is 0, 1 or2;
Y' is O or S;
or a pharmaceutically acceptable salt thereof.

2. A compound according to claim 1 which is
cis- {4-cyano-4-[3-(cis-3-hydroxycyclopentyloxy)-4-methoxyphenyl]cyclohexane-1-carboxylic acid};
cis- {-4-cyano-4-[3-(cis-3-formyloxycyclopentyloxy)-4-methoxyphenyl]cyclohexane-1-carboxylic acid}, or
cis- {-4-cyano-4-[3-(trans-3-hydroxycyclopentyloxy)-4-methoxyphenyl]cyclohexane-1-carboxylic acid}, or a pharmaceutically acceptable salt thereof.

3. A pharmaceutical composition comprising a compound according to any one of claims 1-2 and a pharmaceutically acceptable excipient.

## Patentansprüche

1. Verbindung der Formel I 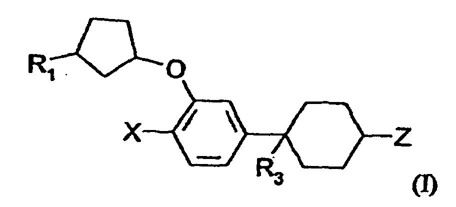 wobei
R₁ gleich -OH oder -OCOH ist;
X gleich -YR₂ ist;
Y gleich O ist und R₂ eine Methylgruppe ist, die mit einem oder mehreren Halogenatomen substituiert ist;
Z gleich -C(O)OH, -C(=Y')R₁₄, -C(=O)OR₁₄, -C(=Y')NR₁₀R₁₄, -C(=NR₁₀)NR₁₀R₁₄ ist;
R₂ eine Methylgruppe oder Ethylgruppe ist, wobei beide, Methylgruppe oder Ethylgruppe, gegebenenfalls mit einem oder mehreren Halogenatomen substituiert sein können;
R₃ gleich -CN oder -C≡CH ist;
R₄ und R₅ unabhängig voneinander ein Wasserstoffatom oder ein C₁₋₂ Alkylrest sind;
R₁₄ ein Wasserstoffatom oder R₇ ist, oder wenn R₁₀ und R₁₄ wie NR₁₀R₁₄ sind, sie zusammen mit dem Stickstoffatom einen 5- bis 7-gliedrigen Ring bilden können, der gegebenenfalls mindestens ein weiteres Heteroatom, ausgewählt aus O, N oder S, umfasst;
R₇ gleich -(CR₄R₅)_{q}R₁₂ oder ein C₁₋₆ Alkykest ist, wobei R₁₂ oder der C₁₋₆ Alkylrest gegebenenfalls ein- oder mehrfach mit C₁₋₂ Alkylresten, die gegebenenfalls substituiert sind mit ein bis drei Resten ausgewählt aus -F, -Br, -Cl, -NO₂, -NR₁₀R₁₁, -C(=O)R₈, -C(=O)OR₈, -OR₈, -CN, -C(=O)NR₁₀R₁₁, -OC(=O)NR₁₀R₁₁, -OC(=O)R₈, -NR₁₀C(=O)NR₁₀R₁₁, -NR₁₀C(=O)R₁₁, -NR₁₀C(=O)OR₉, -NR₁₀C(=O)R₁₃, -C(=NR₁₀)NR₁₀R₁₁, -C(=N-CN)NR₁₀R₁₁, -C(=N-CN)SR₉, -NR₁₀C(=N-CN)NR₁₀R₁₁, -NR₁₀S(=O)₂R₉, -S(O)ₘ'R₉, -NR₁₀C(=O)C(=O)NR₁₀R₁₁, -NR₁₀C(=O)C(=O)R₁₀, einer Thiazolyl-, Imidazolyl-, Oxazolyl-, Pyrazolyl-, Triazolyl- oder Tetrazolylgruppe, substituiert sind;
R₈ ein Wasserstoffatom oder R₉ ist;
R₉ ein C₁₋₄ Alkylrest ist, der gegebenenfalls mit einem bis drei Fluoratomen substituiert ist;
R₁₀ gleich OR₈, ein Wasserstoffatom oder ein C₁₋₄ Alkylrest ist, der gegebenenfalls mit einem bis drei Fluoratomen substituiert ist;
R₁₁ ein Wasserstoffatom oder ein C₁₋₄ Alkylrest ist, der gegebenenfalls mit einem bis drei Fluoratomen substituiert ist; oder wenn R₁₀ und R₁₁ wie NR₁₀R₁₁ sind, sie zusammen mit dem Stickstoffatom einen 5- bis 7-gliedrigen Ring bilden können, der gegebenenfalls mindestens ein weiteres Heteroatom, ausgewählt aus O, N oder S, umfasst;
R₁₂ ein C₃₋₇ Cycloalkylrest, eine 2-Pyridyl-, 3-Pyridyl-, 4-Pyridyl-, Pyrimidyl-, Pyrazolyl-, 1-Imidazolyl-, 2-Imidazolyl-, Thiazolyl-, Triazolyl-, Pyrrolyl-, Piperazinyl-, Piperidinyl-, Morpholinyl-, Furanyl-, 2-Thienyl-, 3-Thienyl-, 4-Thiazolyl-, 5-Thiazolyl-, Chinolinyl-, Naphthyl- oder Phenylgruppe ist;
R₁₃ ein heterocyclischer Ring ist, ausgewählt aus einer Oxazolidinyl-, Oxazolyl-, Thiazolyl-, Pyrazolyl-, Triazolyl-, Tetrazolyl-, Imidazolyl-, Imidazolidinyl-, Thiazolidinyl-, Isoxazolyl-, Oxadiazolyl- oder Thiadiazolylgruppe, wobei R₁₃ über ein Kohlenstoffatom des heterocyclischen Rings an eine Verbindung der Formel (I) gebunden ist, und wobei jeder heterocyclische Ring nicht substituiert oder mit einem oder zwei C₁₋₂ Alkylresten substituiert sein kann;
m' gleich 0, 1 oder 2 ist;
q gleich 0, 1 oder 2 ist;
Y' gleich O oder S ist;
oder ein pharmazeutisch verträgliches Salz davon.

2. Verbindung gemäß Anspruch 1, nämlich
cis-{4-Cyano-4-[3-(cis-3-hydroxycyclopentyloxy)-4-methoxyphenyl]cyclohexan-1-carbonsäure};
cis-{4-Cyano-4-[3-(cis-3-formyloxycyclopentyloxy)-4-methoxyphenyl]cyclohexan-1-carbonsäure}; oder
cis-{4-Cyano-4-[3-(trans-3-hydroxycyclopentyloxy)-4-methoxyphenyl]cyclohexan-1-carbonsäure},
oder ein pharmazeutisch verträgliches Salz davon.

3. Arzneimittel umfassend eine Verbindung gemäß einem der Ansprüche 1 oder 2 und einen pharmazeutisch verträglichen Exzipienten.

## Revendications

1. Composé de formule I 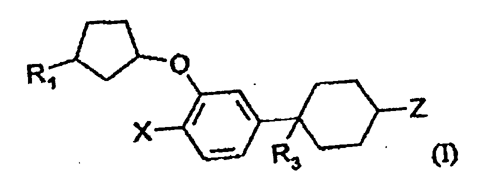 dans laquelle :
R₁ représente un groupe OH ou -OCOH ;
X représente un groupe YR₂ ;
Y représente O et R₂ représente un groupe méthyle substitué avec un ou plusieurs halogènes ;
Z représente un groupe -C(O)OH, C(=Y')R₁₄, C(=O)OR₁₄, C(=Y')NR₁₀R₁₄ ou C(=NR₁₀)NR₁₀R₁₄ ;
R₂ représente un groupe méthyle ou éthyle, le groupe méthyle ou éthyle pouvant être facultativement substitué avec un ou plusieurs halogènes ;
R₃ représente un groupe CN ou -C≡CH ;
R₄ et R₅ représentent, indépendamment, l'hydrogène ou un groupe alkyle en C₁ ou C₂ ;
R₁₄ représente H ou un groupe R₇ ou bien, lorsque R₁₀ et R₁₄ sont sous forme d'un groupe NR₁₀R₁₄, ils peuvent, conjointement avec l'atome d'azote, former un noyau penta- à heptagonal contenant facultativement au moins un hétéroatome supplémentaire choisi entre O, N et S ;
R₇ représente un groupe -(CR₄R₅)_{q}R₁₂ ou alkyle en C₁ à C₆, dans lequel le groupe R₁₂ ou alkyle en C₁ à C₆ est facultativement substitué une ou plusieurs fois avec un substituant alkyle en C₁ ou C₂ facultativement substitué avec un à trois groupes choisis entre des groupes -F, -Br, -Cl, -NO₂, -NR₁₀R₁₁, -C(=O)R₈, -C(=O)OR₈, -OR₈, -CN, -C(=O)NR₁₀R₁₁, -OC(=O)NR₁₀R₁₁, -OC(=O)R₈, -NR₁₀C(=O)NR₁₀R₁₁, -NR₁₀C(=O)R₁₁, -NR₁₀C(=O)OR₉, -NR₁₀C(=O)R₁₃, -C (=NR₁₀)NR₁₀R₁₁, -C (=N-CN) NR₁₀R₁₁, -C (=N-CN) SR₉, -NR₁₀C(=N-CN)NR₁₀R₁₁, -NR₁₀S(=O)₂R₉, -S(O)ₘ'R₉, -NR₁₀C(=O)C(=O)NR₁₀R₁₁, -NR₁₀C(=O)C(=O)R₁₀, thiazoyle, imidazolyle, oxazolyle, pyrazolyle, triazolyle et tétrazolyle ;
R₈ représente -H ou un groupe R₉ ;
R₉ représente un groupe alkyle en C₁ à C₄ facultativement substitué avec un à trois substituants -F ;
R₁₀ représente un groupe OR₈, l'hydrogène, ou un groupe alkyle en C₁ à C₄ facultativement substitué avec un à trois atomes de fluor ;
R₁₁ représente -H ou un groupe alkyle en C₁ à C₄ facultativement substitué avec un à trois -F ; ou bien, lorsque R₁₀ et R₁₁ sont sous forme d'un groupe NR₁₀R₁₁, ils peuvent, conjointement avec l'atome d'azote, former un noyau penta- à heptagonal contenant facultativement au moins un hétéroatome supplémentaire choisi entre O, N et S ;
R₁₂ représente un groupe cycloalkyle en C₃ à C₇, 2-pyridyle, 3-pyridyle, 4-pyridyle, pyrimidyle, pyrazolyle, 1-imidazolyle, 2-imidazolyle, thiazolyle, triazolyle, pyrrolyle, pipérazinyle, pipéridinyle, morpholinyle, furannyle, 2-thiényle, 3-thiényle, 4-thiazolyle, 5-thiazolyle, quinolinyle, naphtyle ou phényle ;
R₁₃ représente un noyau hétérocyclique choisi entre les noyaux oxazolidinyle, oxazolyle, thiazolyle, pyrazolyle, triazolyle, tétrazolyle, imidazolyle, imidazolidinyle, thiazolidinyle, isoxazolyle, oxadiazolyle et thiadiazolyle, où R₁₃ est appendu à un composé de formule (I) par un atome de carbone du noyau hétérocyclique, et où chaque noyau hétérocyclique peut être non substitué ou substitué avec un ou deux groupes alkyle en C₁ ou C₂ ;
m' est égal à 0, 1 ou 2 ;
q est égal à 0, 1 ou 2 ;
Y' représente O ou S ;
ou un de ses sels pharmaceutiquement acceptables.

2. Composé suivant la revendication 1, qui est
l'acide cis-{4-cyano-4-[3-(cis-3-hydroxycyclopentyloxy)-4-méthoxyphényl]cyclohexane-1-carboxylique} ;
l'acide cis{4-cyano-4-[3-(cis-3-formyloxycyclopentyloxy)-4-méthoxyphényl]cyclohexane-1-carboxylique}, ou
l'acide cis{4-cyano-4-[3-(trans-3-hydroxycyclopentyloxy)-4-méthoxyphényl]cyclohexane-1-carboxylique}, ou un de ses sels pharmaceutiquement acceptables.

3. Composition pharmaceutique comprenant un composé suivant l'une quelconque des revendication 1 et 2 et un excipient pharmaceutiquement acceptable.
